(19) **Europäisches Patentamt**

**European Patent Office**

**Office européen des brevets**

(11) **EP 0 863 198 B1**

(12) **FASCICULE DE BREVET EUROPEEN**

(45) Date de publication et mention
de la délivrance du brevet:
**04.08.2004 Bulletin 2004/32**

(51) Int Cl.[7]: **C10G 45/64**, B01J 29/70

(21) Numéro de dépôt: **98400386.3**

(22) Date de dépôt: **18.02.1998**

(54) **Catalyseur à base de tamis moléculaire et procédé d'hydroisomérisation sélective de paraffines longues linéaires et/ou peu ramifiées avec ce catalyseur**

Auf einem Molekularsieb basierender Katalysator und Verfahren zur selektiven Hydroisomerisierung von langen geradkettigen und/oder verzweigten Paraffinen

Molecular sieve-based catalyst and process for the selective hydroisomeration of long straight chain and/or branched chain paraffins using this catalyst

(84) Etats contractants désignés:
**DE ES GB IT NL**

(30) Priorité: **05.03.1997 FR 9702598**

(43) Date de publication de la demande:
**09.09.1998 Bulletin 1998/37**

(73) Titulaire: **Institut Français du Pétrole**
**92500 Rueil Malmaison (FR)**

(72) Inventeurs:
• **Marcilly, Christian**
**78800 Houilles (FR)**
• **Benazzi, Eric**
**78400 Chatou (FR)**
• **George-Marchal, Nathalie**
**75005 Paris (FR)**

(56) Documents cités:
**EP-A- 0 065 400** **EP-A- 0 103 981**
**WO-A-92/01657** **WO-A-97/09397**

Remarques:
Le dossier contient des informations techniques présentées postérieurement au dépôt de la demande et ne figurant pas dans le présent fascicule.

## Description

La présente invention est un perfectionnement aux demandes de brevet FR-95/10.425 et FR -95/10.424 déposées le 6 septembre 1995 et elle concerne un catalyseur et un procédé d'hydroisomérisation sélective de paraffines, linéaires et/ou peu ramifiées, longues (plus de 10 atomes de carbone), en particulier pour convertir, avec un bon rendement, des charges possédant des points d'écoulement élevés en au moins une coupe présentant un point d'écoulement bas et un haut indice de viscosité.

## Art antérieur

Les lubrifiants de haute qualité sont d'une importance primordiale pour le bon fonctionnement des machines modernes, des automobiles, et des camions. Cependant, la quantité de paraffines issues directement du pétrole, non traitées, et possédant les propriétés adéquates pour constituer des bons lubrifiants est très faible par rapport à la demande croissante dans ce secteur.

Le traitement des fractions pétrolières lourdes à fortes teneurs en paraffines linéaires ou peu ramifiées est nécessaire afin d'obtenir des huiles de base de bonne qualité et ce avec les meilleurs rendements possibles, par une opération de déparaffinage qui vise à éliminer les paraffines linéaires ou très peu branchées, des charges qui seront ensuite utilisées en tant que huiles de base ou en tant que kérosène ou carburéacteur (jet fuel).

En effet, les paraffines de haut poids moléculaire qui sont linéaires ou très faiblement branchées et qui sont présentes dans les huiles ou dans le kérosène ou carburéacteur conduisent à des points d'écoulement hauts et donc à des phénomènes de figeage pour des utilisations à basse température. Afin de diminuer les valeurs des points d'écoulement, ces paraffines linéaires pas ou très peu branchées doivent être entièrement ou partiellement éliminées.

Cette opération de déparaffinage peut s'effectuer par extraction par des solvants tels que le propane ou la méthyl-éthyl cétone, on parle alors de déparaffinage au propane ou à la méthyl éthyl-cétone (MEK). Cependant, ces techniques sont coûteuses, longues et pas toujours aisées à mettre en oeuvre.

Le déparaffinage catalytique ainsi dénommé par opposition au déparaffinage par solvant est plus économique et permet d'obtenir des produits possédant les propriétés physico-chimiques désirées. Ceci est obtenu par un craquage sélectif des chaînes paraffiniques linéaires les plus longues qui conduit à la formation de composés de poids moléculaire plus faible dont une partie peut être éliminée par distillation.

Compte tenu de leur sélectivité de forme les zéolithes sont parmi les catalyseurs de déparaffinage les plus utilisés. L'idée qui prévaut à leur utilisation est qu'il existe des structures zéolithiques dont les ouvertures de pores sont telles qu'elles permettent l'entrée dans leur microporosité des paraffines linéaires longues ou très peu branchées mais en excluent les paraffines ramifiées, les napthènes et les aromatiques. Ce phénomène conduit ainsi à un craquage sélectif des paraffines linéaires ou très peu branchées.

Des catalyseurs à base de zéolithes ayant des tailles de pores intermédiaires telles que les ZSM-5, ZSM-11, ZSM-12, ZSM-22, ZSM-23, ZSM-35 et ZSM-38 ont été décrits pour leur utilisation dans les procédés de déparaffinage catalytique par craquage.

Les procédés utilisant ces zéolithes permettent le déparaffinage par craquage de charges contenant des quantités de paraffines linéaires ou très peu branchées inférieures à 50% poids. Cependant, pour des charges contenant des quantités supérieures de ces composés il est apparu que leur craquage conduit à la formation de quantités importantes de produits de poids moléculaires plus faibles, tels que du butane, propane, éthane et méthane, ce qui réduit considérablement le rendement en produits recherchés.

Pour pallier à ces inconvénients, la demanderesse a porté ses efforts de recherche sur la mise au point de catalyseurs (de préférence non ZSM) permettant une isomérisation de ces composés.

De nombreux brevets existent dans ce domaine par exemple, le brevet WO 92/01657 décrit et revendique un procédé de déparaffinage de charges avec réaction d'isomérisation en présence d'un métal du groupe VIII, d'une pression d'hydrogène comprise entre 100 KPa et 21000 KPa et d'un catalyseur dont l'ouverture de pores est comprise entre 0,48 nm et 0,71 nm et dont la taille des cristallites est inférieure à 0,5 μm. Ce catalyseur conduit à des performances améliorées en termes de rendement par rapport aux catalyseurs de l'art antérieur.

## Objet de l'invention

L'invention a pour objet un catalyseur comprenant une matrice, au moins 5% d'au moins un élément hydro-déshydrogénant choisi dans le groupe formé par les métaux non nobles du GVIII, les métaux de GVIB, et le niobium, et au moins un tamis moléculaire ayant un réseau poreux mono ou bidimensionnel, dont les ouvertures de pores accessibles sont délimitées par 10 atomes d'oxygène, et la distance dite largeur de pont entre lesdits pores étant inférieure à 0,70 nm ledit tamis contenant des atomes de bore, gallium et/ou zinc dans le réseau zéolithique, et la zéolite est telle que le catalyseur constitué de la zéolite et de 0,5% pds de platine soumis au test standard d'isomérisation du n-heptadécane présente, pour une conversion de 95%, une sélectivité en produits isomérisés d'au moins 70%.

L'invention a également pour objet un procédé d'hydroisomérisation sélective de composés ayant au moins une chaîne n-alcane à plus de 10 atomes de carbone, dans lequel ledit composé à traiter est mis au contact du catalyseur selon l'invention

Avantageusement ce procédé permet de convertir une charge possédant un haut point d'écoulement en un mélange possédant un point d'écoulement plus bas et un haut indice de viscosité.

La charge comprend, entre autre, des paraffines linéaires et/ou peu ramifiées comportant au moins 10 atomes de carbone, de préférence de 15 à 50 atomes de carbone et avantageusement de 15 à 40 atomes de carbone.

Le procédé comprend l'utilisation d'un catalyseur comportant au moins un tamis moléculaire possédant au moins un type de pores dont l'ouverture est délimitée par 10 atomes d'oxygène et qui sont les pores accessibles de l'extérieur les plus grands présents dans la structure. Le réseau poreux de la zéolithe est mono ou bidimensionnel, et de préférence monodimensionnel.

La largeur de pont entre deux ouvertures de pores (à 10 atomes d'oxygène), telle que définie précédemment, est inférieure à 0,70 nm (1nm =$10^{-9}$ m) de préférence comprise entre 0,50 nm et 0,68 nm, de manière encore plus préférée entre 0,52 nm et 0,65 nm. De préférence, les tailles de cristallites sont inférieures à 2 µm (1µm = $10^{-6}$ m), avantageusement inférieure à 1µm et de préférence à 0,4 µm.

La zéolite est telle que le catalyseur constitué de la zéolite et de 0,5 % pds de platine, conduise, pour une conversion de l'ordre de 95 % en poids de n-heptadécane (n-C17), à une sélectivité en produits isomérisés supérieure ou égale à 70% et de préférence d'au moins 80%, dans les conditions d'un test standard d'isomérisation du n-C17 (TSI) qui seront définies ci-après. Les produits isomérisés contiennent généralement entre environ 65 et 80% pds de produits monobranchés et entre environ 20 et 35 % pds de produits multibranchés, essentiellement des dibranchés. On entend par produits monobranchés des paraffines linéaires comportant un seul groupe méthyl, et par produits bibranchés des paraffines linéaires comportant 2 groupes méthyles qui ne sont pas portés sur le même atome de carbone. Par extension on définit ainsi les multibranchés. L'hydroisomérisation est ainsi sélective.

D'autre part le catalyseur comporte au moins une fonction hydro-déshydrogénante amenée par au moins un élément hydro-déshydrogénant choisi dans le groupe formé par les métaux non nobles du groupe VIII, les métaux du groupe VI B, le niobium, et la réaction est réalisée dans les conditions décrites ci-après.

La demanderesse a en effet découvert de façon surprenante que l'un des facteurs déterminant pour l'obtention de fortes sélectivités en produits isomérisés est l'utilisation de tamis moléculaires caractérisé en ce que

- l'ouverture des pores les plus grands est délimitée par 10 atomes d'oxygène,
- la largeur de pont doit être inférieure à 0,70 nm, de préférence comprise entre 0,50 nm et 0,68 nm, de manière préférée entre 0,52 nm et 0,65 nm.

Ce dernier point en particulier est en contradiction avec ce qui est revendiqué dans les brevets précédemment cités dans l'art antérieur tel que WO 92/01657 qui donne comme moyen essentiel une gamme de taille de pores pour l'obtention de bons rendements en produits isomérisés.

La mesure de largeur de pont est réalisée en utilisant un outil de graphisme et de modélisation moléculaire tel que Hyperchem ou Biosym, qui permettent de construire la surface des tamis moléculaires en question et, en tenant compte des rayons ioniques des éléments présents dans la charpente du tamis, de mesurer la largeur de pont.

L'utilisation de tels tamis moléculaires selon l'invention dans les conditions décrites ci-dessus permet, notamment, la production de produits à faible point d'écoulement et haut indice de viscosité avec de bons rendements.

## Description détaillée de l'invention

Les tamis moléculaires selon l'invention qui peuvent être utilisés pour les réactions d'isomérisation des hydrocarbures paraffiniques linéaires ou peu branchés sont des zéolithes, aluminosilicates cristallisés tels que Theta-1, NU-10, NU-23, EU-13, dont le rapport Si/Al est celui qui convient le mieux pour l'application désirée et qui contient des atomes bore, gallium et/ou zinc dans le réseau zéolitique. Parmi les zéolithes selon l'invention figurent aussi la zéolithe NU-87 qui possède certes des pores délimités par 10 et 12 atomes d'oxygène mais dont l'accessibilité à ces derniers se fait par les ouvertures de pores à 10 atomes d'oxygène. Les dérivés des zéolithes précédemment décrites comportant au moins un hétéroatome dans le réseau zéolithique et tel que B, Fe, Ga, Zn, sont aussi compris dans les termes de l'invention.

La zéolithe NU-10 employée dans le procédé selon l'invention ainsi que son mode de synthèse sont décrits dans le brevet EP-A-77624. Cette zéolithe NU-10 est caractérisée par un tableau de diffraction des rayons X qui est le suivant :

Tableau de diffraction des rayons X de la zéolithe NU-10

| d(A) | I/Io |
|---|---|
| 10,95 ± 0,25 | m à F |
| 8,80 ± 0,14 | f à m |
| 6,99 ± 0,14 | f à m |
| 5,41 ± 0,10 | f |
| 4,57 ± 0,09 | f |
| 4,38 ± 0,08 | TF |
| 3,69 ± 0,07 | TF |
| 3,63 ± 0,07 | TF |
| 3,48 ± 0,06 | m à F |
| 3,36 ± 0,06 | f |
| 3,31 ± 0,05 | f |
| 2,78 ± 0,05 | f |
| 2,53 ± 0,04 | m |
| 2,44 ± 0,04 | f |
| 2,37 ± 0,03 | f |
| 1,88 ± 0,02 | f |

f = faible (I/Io compris entre 0 et 20, m=moyen (I/Io compris entre 20 et 40), F=Fort (I/Io compris entre 40 et 60), TF=Trés Fort (I/Io compris entre 60 et 100).

La zéolite Nu-10 présente un rapport atomique Si/Al compris entre 8 et 1000.

On a pu observer que, les zéolites entrant dans les catalyseurs selon l'invention sont caractérisées par un test catalytique dit test standard d'isomérisation (TSI) du n-heptadécane pur qui est réalisé sous une pression partielle de 150 kPa d'hydrogène et une pression partielle de n-C17 de 0,5 kPa soit une pression totale de 150,5 kPa en lit fixe et avec un débit de n-C17 constant de 15,4 ml/h et une masse de catalyseur de 0,5 g. La réaction est réalisée en flux descendant. Le taux de conversion est réglé par la température à laquelle se déroule la réaction. Le catalyseur soumis au dit test est constitué de zéolithe pure pastillée et de 0,5% poids de platine.

Dans ces conditions un tamis moléculaire selon l'invention doit conduire, pour un taux de conversion du n-C17 de l'ordre de 95% poids (le taux de conversion est réglé par la température), à une sélectivité en produits isomérisés supérieure ou égale à 70% poids et de préférence d'au moins 80% poids.

La sélectivité en isomérisation du test standard d'isomérisation du n-C17 ($TSI_{n-C17}$) est définie comme suit :

$$\text{Sélectivité isomérisation (\%) =}$$

$$\frac{\text{masse de produits isomérisés } C_{17} \text{ (monobranchés + multibranchés) dans la recette}}{\text{masse de produits isomérisés en } C_{17} \text{ dans la recette + masse de produits } C_{17}^{-} \text{ dans la recette}} \times 100$$

à une conversion du n-C17 de l'ordre de 95%.

Les produits en $C_{17}^{-}$ sont les composés qui possèdent moins de 17 atomes de carbone, quelque soit leur degré de ramification.

Le tamis contient généralement au moins un élément hydro-déshydrogénant qui est introduit dans le tamis moléculaire par exemple par imprégnation à sec, par échange ionique ou toute autre méthode connue de l'homme du métier.

La teneur en métal (métaux) hydro-déshydrogénant(s) ainsi introduit, exprimée en % poids par rapport à la masse de tamis moléculaire engagée, est d'au moins 5%.

La fonction hydro-deshydrogénante est assurée de préférence par au moins un métal ou composé de métal du groupe VIII tels que le nickel et le cobalt notamment. On peut utiliser une combinaison d'au moins un métal ou composé de métal du groupe VI (notamment le molybdène ou le tungstène) et d'au moins un métal ou composé de

4

métal du groupe VIII (notamment le cobalt ou le nickel) de la classification périodique des éléments. La concentration totale en oxydes de métaux des groupes VI et/ou VIII est comprise entre 6,4 et 40 % en poids et de préférence entre 7 et 40 %, avantageusement entre 8 et 40%, voire 10 à 40% et mieux 10-30% en poids, et le rapport pondéral exprimé en oxyde métallique de métal (ou métaux) du groupe VI sur métal (ou métaux) du groupe VIII est compris entre 1,25 et 20 et de préférence entre 2 et 10. De plus, ce catalyseur peut contenir du phosphore. La teneur en phosphore, exprimée en concentration en oxyde de phosphore $P_2O_5$, est généralement inférieure à 15 % poids et de préférence inférieure à 10 % en poids.

La fonction hydrogénante telle qu'elle a été définie précédemment (métaux du groupe VIII ou association d'oxydes de métaux des groupes VI et VIII) peut être introduite dans le catalyseur à divers niveaux de la préparation et de diverses manières.

Elle peut être introduite en partie seulement (cas des associations d'oxydes de métaux des groupes VI et VIII) ou en totalité au moment du malaxage du tamis moléculaire selon l'invention, avec le gel d'oxyde choisi comme matrice. Elle peut être introduite par une ou plusieurs opérations d'échange ionique sur le support calciné constitué du tamis moléculaire selon l'invention dispersé dans la matrice choisie, à l'aide de solutions contenant les sels précurseurs des métaux choisis lorsque ceux-ci appartiennent au groupe VIII. Elle peut être introduite par une ou plusieurs opérations d'imprégnation du support mis en forme et calciné, par une solution des précurseurs des oxydes des métaux des groupes VIII (notamment le cobalt et le nickel) lorsque les précurseurs des oxydes des métaux du groupe VI (notamment le molybdène ou le tungstène) ont été préalablement introduits au moment du malaxage du support. Elle peut enfin être introduite par une ou plusieurs opérations d'imprégnation du support calciné constitué d'un tamis moléculaire selon l'invention et de la matrice, par des solutions contenant les précurseurs des oxydes de métaux des groupes VI et/ou VIII, les précurseurs des oxydes de métaux de groupe VIII étant de préférence introduits après ceux du groupe VI ou en même temps que ces derniers.

Dans le cas où les oxydes des métaux sont introduits en plusieurs imprégnations des sels précurseurs correspondants, une étape de calcination intermédiaire du catalyseur devra être effectuée à une température comprise entre 250 et 600 °C.

L'imprégnation du molybdène peut être facilitée par ajout d'acide phosphorique dans les solutions de paramolybdate d'ammonium.

Le mélange est ensuite mis en forme, par exemple par extrusion au travers d'une filière. La teneur en tamis moléculaire du mélange ainsi obtenu est généralement comprise entre 0,5 et 99,9% et avantageusement comprise entre 10 et 90% en poids par rapport au mélange (tamis moléculaire + matrice), et de préférence entre 20-70%.

Dans la suite du texte on désignera par le terme support le mélange tamis moléculaire + matrice.

La mise en forme peut être réalisée avec d'autres matrices que l'alumine, telles que par exemple la magnésie, les silice-alumines amorphes, les argiles naturelles (kaolin, bentonite, sepiolite, attapulgite) et par d'autres techniques que l'extrusion, telles que le pastillage ou la dragéification.

Le dépôt du dernier métal est suivi en général d'une calcination sous air ou oxygène, usuellement entre 300 et 600°C durant 0,5 à 10 heures, de préférence entre 350°C et 550°C durant 1 à 4 heures.

Il est ensuite généralement procédé à une sulfuration du catalyseur avant mise en contact avec la charge, par toute méthode connue de l'homme du métier. Le catalyseur contient donc avantageusement du soufre.

Les charges qui peuvent être traitées selon le procédé selon l'invention sont avantageusement des fractions possédant des points d'écoulement relativement hauts dont on désire diminuer la valeur.

Le procédé selon l'invention peut être utilisé pour traiter des charges variées allant de fractions relativement légères telles que les kérosènes et carburéacteurs jusqu'à des charges possédant des points d'ébullition plus élevés telles que les distillats moyens, les résidus sous vide, les gazoles, les distillats moyens issus du FCC (LCO et HCO) et les résidus d'hydrocraquage.

La charge à traiter est dans la majeur partie des cas une coupe C10+ de point d'ébullition initial supérieur à environ 175°C, ou une coupe C20+ à point d'ébullition initial supérieur à 315°C, et de préférence une coupe lourde à point d'ébullition initial d'au moins 380°C. Le procédé selon l'invention est particulièrement adapté pour traiter des distillats paraffiniques tels que les distillats moyens qui englobent les gazoles, les kérosènes, les carburéacteurs et toutes autres fractions dont le point d'écoulement et la viscosité doivent être adaptés pour rentrer dans le cadre des spécifications Les charges qui peuvent être traitées selon le procédé de l'invention peuvent contenir des paraffines, des oléfines, des naphtènes, des aromatiques et aussi des hétérocycles et avec une proportion importante de n-paraffines de haut poids moléculaire et de paraffines très peu branchées également de haut poids moléculaire.

La réaction peut être conduite de façon à ce que le taux des réactions de craquage reste suffisamment faible pour rendre le procédé économiquement viable. Le taux des réactions de craquage est généralement inférieur à 20% pds.

Des charges typiques qui peuvent être traitées avantageusement selon l'invention possèdent en général un point d'écoulement au dessus de 0°C et plus couramment au-dessus de 15°C. Les produits résultant du traitement selon le procédé ont des points d'écoulement inférieurs à 0°C et de préférence inférieurs à environ -10°C.

Ces charges possèdent des teneurs en n-paraffines, (n-alcanes) à plus de 10 atomes de carbone, de haut poids moléculaire et de paraffines, à plus de 10 atomes de carbone, très peu branchées également de haut poids moléculaire, supérieures à 30% et jusqu'à environ 90%, voire dans certains cas supérieures à 90% poids. Le procédé est particulièrement intéressant lorsque cette proportion est d'au moins 60% poids.

On peut citer comme exemples d'autres charges traitables selon l'invention et à titre non limitatifs, les bases pour huiles lubrifiantes, les paraffines de synthèse issues du procédé Fischer-Tropsch, les polyalphaoléfines à haut point d'écoulement, les huiles de synthèse etc... Le procédé peut également s'appliquer à d'autres composés contenant une chaîne n-alcane tels que définis précédemment, par exemple des composés n-alkylcycloalcanes, ou comportant au moins un groupe aromatique.

Les conditions opératoires dans lesquelles s'opère l'hydroisomérisation selon le procédé de l'invention sont les suivantes:

- la température de réaction est comprise entre 170 et 500°C et de préférence entre 180 et 450°C, avantageusement 180-400°C,
- la pression est comprise entre 1 et 250 bar et de préférence entre 10 et 200 bar,
- la vitesse volumique horaire (vvh exprimée en volume de charge injectée par unité de volume de catalyseur et par heure) est comprise entre environ 0,05 et environ 100 et de préférence entre environ 0.1 et environ 30 h-1.

Le contact entre la charge et le catalyseur est réalisé en présence d'hydrogène. Le taux d'hydrogène utilisé et exprimé en litre d'hydrogène par litre de charge est compris entre 50 et environ 2000 litres d'hydrogène par litre de charge et de préférence entre 100 et 1500 litres d'hydrogène par litre de charge.

La charge à traiter possède de préférence une teneur en composés azotés inférieure à environ 200 ppm poids et de préférence inférieure à 100 ppm poids. La teneur en soufre est inférieure à 1000 ppm poids, de préférence inférieure à 500 ppm et de manière encore plus préférée inférieure à 200 ppm poids. La teneur en métaux de la charge, tels que Ni ou V, est extrêmement réduite, c'est à dire inférieure à 50 ppm poids, de manière préférée inférieure à 10 ppm poids et de manière encore plus préférée inférieure à 2 ppm poids.

Les composés obtenus par ledit procédé selon l'invention sont essentiellement monobranchés, dibranchés et multibranchés avec des groupes méthyles. Par exemple, dans le cas d'une charge constituée de n-heptadécane pur (n-C17), on a obtenu de façon sélective des composés méthylhexadécane, majoritairement le 2-méthylhexadécane, ainsi que les composés dibranchés 2,7-; 2,8-; 2,9; 2,10- et 2,11-diméthylpentadécane. L'ensemble des produits isomérisés représente plus de 70% poids des produits obtenus à une conversion de 95% poids. On observe que les atomes de carbone isomérisés sont séparés d'une distance au moins égale à la largeur de pont.

Les exemples qui suivent illustrent l'invention sans toutefois en limiter la portée. Ils sont donnés pour une charge constituée de n-heptadécane (test standard d'isomérisation, TSI) ou un résidu d'hydrocraquage.

## Exemple 1: Catalyseur C1 comparatif

La matière première utilisée est une zéolithe NU-10 sous forme H, qui possède un rapport Si/Al global d'environ 30, une ouverture de pores délimitée par 10 atomes d'oxygène et une largeur de pont, c'est à dire une distance entre deux ouvertures de pores de 0,55 nm. Les cristallites de la zéolithe NU-10 se présentent sous forme d'aiguilles dont la longueur est inférieur à 1 μm et la section est comprise entre 0,1 et 0,4 μm.

La zéolithe Nu-10 est malaxée avec de l'alumine de type SB3 fournie par la société Condéa. La pâte malaxée est ensuite extrudée à travers une filière de diamètre 1,4 mm. Les extrudés sont imprégnés à sec par une solution d'un mélange d'heptamolybdate d'ammonium, de nitrate de nickel et d'acide orthophosphorique, et enfin calcinés sous air à 550 °C in-situ dans le réacteur. Les teneurs pondérales en oxydes actifs sont les suivantes (par rapport au catalyseur) :

5,2 % en poids d'oxyde de phosphore $P_{2O5}$
15,2 % en poids d'oxyde de molybdène $MoO_3$
2,8 % en poids d'oxyde de nickel NiO

La teneur en zéolithe NU-10 dans l'ensemble du catalyseur est de 60%.

Evaluation du catalyseur en hydroisomérisation d'un résidu d'hydrocraquage issu d'un distillat sous vide.

Les caractéristiques de la charge sont les suivantes :

| | |
|---|---|
| Teneur en soufre (ppm poids) | 98 |
| Teneur en azote (ppm poids) | 12 |

(suite)

| | |
|---|---|
| Point d'écoulement (°C) | +36 |
| Point initial | 369 |
| 5% | 422 |
| 10% | 438 |
| 50% | 477 |
| 90% | 489 |
| 95% | 524 |
| Point final | 549 |

L'unité de test catalytique comprend un réacteur en lit fixe, à circulation ascendante de la charge ("up-flow"), dans lequel est introduit 80 ml de catalyseur. Chacun des catalyseurs est sulfuré par un mélange n-hexane/DMDS + aniline jusqu'à 350 °C. La pression totale est de 12 MPa, le débit d'hydrogène est de 1000 litres d'hydrogène gazeux par litre de charge injectée, la vitesse volumique horaire est de 1,0 h-1.

La réaction a lieu à 300 °C, sous une pression totale de 12 MPa, une vitesse volumique horaire 1,1h-1 et un débit d'hydrogène de 1000l H2 par litre de charge.

Les caractéristiques de l'huile après hydroisomérisation sont reportées dans le tableau ci-après.

| | |
|---|---|
| Indice de viscosité VI | 112 |
| Point d'écoulement (°C) | -18 |
| Rendement Huile/charge (% poids) | 77 |

Cet exemple montre tout l'intérêt qu'il y a à utiliser un catalyseur selon l'invention, qui permet d'abaisser le point d'écoulement de la charge initiale, dans ce cas un résidu d'hydrocraquage, tout en conservant un haut indice de viscosité (VI).

La présente invention a été illustrée dans le but d'obtenir une huile, mais d'autres objectifs peuvent être atteints. Généralement, l'invention sera employée pour obtenir des branchements multiples et localisés.

## Revendications

1. Catalyseur comprenant une matrice, au moins 5% pds d'au moins un élément hydro-déshydrogénant choisi dans le groupe formé par les métaux non nobles du GVIII, les métaux de GVIB, et le niobium, et au moins un tamis moléculaire ayant un réseau poreux mono ou bidimensionnel, dont les ouvertures de pores accessibles sont délimitées par 10 atomes d'oxygène, et la distance dite largeur de pont entre lesdits pores étant inférieure à 0,70 nm ledit tamis étant choisi dans le groupe formé par NU-10, NU-23, NU-87, Theta-1, EU-13, et ledit tamis contenant des atomes de bore, gallium et/ou zinc dans le réseau zéolithique, et la zéolite est telle que le catalyseur constitué de la zéolite et de 0,5% pds de platine, soumis au test standard d'isomérisation du n-heptadécane présente, pour une conversion de 95%, une sélectivité en produits isomérisés d'au moins 70%.

2. Catalyseur selon la revendication 1, comprenant également du phosphore.

3. Catalyseur selon l'une des revendications 1 à 2, contenant également du soufre.

4. Procédé d'hydroisomérisation sélective de composés ayant au moins une chaîne n-alcane à plus de 10 atomes de carbone dans lequel ledit composé est mis au contact d'un catalyseur selon l'une des revendications 1 à 3.

5. Procédé selon la revendication 4, dans lequel la pression est comprise entre 1 et 250 bars, la température entre 170 et 500°C, la vitesse volumique horaire entre 0,05 et 100 h$^{-1}$, et le taux d'hydrogène entre 50 et 2000 l d'hydrogène/l de charge.

6. Procédé selon l'une des revendications 4 à 5, dans lequel le composé à traiter est choisi dans le groupe formé par les n-alcanes, les n-alkylcycloalcanes et les composés comportant au moins un groupe aromatique.

**7.** Procédé selon l'une des revendications 4 à 6, dans lequel le composé à traiter est présent dans une charge à point d'ébullition initial supérieur à 175°C.

**8.** Procédé selon l'une des revendications 4 à 6, dans lequel le composé à traiter est présent dans une charge à point d'ébullition initial d'au moins 380°C.

**9.** Procédé selon l'une des revendications 4 à 8, dans lequel le composé à traiter est présent dans une charge hydrocarbonée choisie dans le groupe formé par les distillats moyens, les résidus sous vide, les résidus d'hydrocraquage, les paraffines issues du procédé Fischer-Tropsch, les huiles de synthèse, les coupes gasoils, les distillats moyens issues du FCC, les bases pour huiles, les polyalphaoléfines.

**Patentansprüche**

**1.** Katalysator, umfassend eine Matrix, wenigstens 5 Gew. -% wenigstens eines hydrierend-dehydrierenden Elementes ausgewählt aus der Gruppe gebildet durch die unedlen Metalle der Gruppe VIII, Metalle der Gruppe VIB, Niobium und wenigstens ein Molekularsieb mit einem mono- oder bidimensionellen Porennetz, dessen zugängliche Porenöffnung durch 10 Sauerstoffatome begrenzt ist, und die Breite der Brücke zwischen diesen Poren kleiner als 0,70 nm ist, wobei das Molsieb ausgewählt ist aus der Gruppe gebildet durch NU-10, NU-23, NU-87, Theta-1, EU-13, und wobei das Molsieb Bor-, Galliumund/oder Zinkatome im zeolithischen Porennetz enthält, und der Zeolith, ebenso wie der aus Zeolith und 0,5 Gew.-% Platin bestehende Katalysator, unterzogen einem n-Heptadekan- Standard- Isomerisations- Test bei einer Konversion von 95% eine Selektivität bei isomerisierten Produkten von wenigstens 70% zeigt.

**2.** Katalysator gemäß Anspruch 1, umfassend gleichermaßen Phosphor.

**3.** Anspruch nach einem der Ansprüche 1 oder 2, enthaltend gleichermaßen Schwefel.

**4.** Verfahren zur selektiven Hydroisomerisation von Verbindungen mit wenigstens einer n-Alkyl-Kette mit mehr als 10 Kohlenstoffatomen, bei dem diese Verbindung mit einem Katalysator gemäß einem der Ansprüche 1 bis 3 in Kontakt gebracht wird.

**5.** Verfahren gemäß Anspruch 4, bei dem der Druck zwischen 1 und 250 bar beträgt, die Temperatur zwischen 179 und 500°C liegt, die stündliche Volumengeschwindigkeit zwischen 0,05 und 100h$^{-1}$ liegt, und die Wasserstoffrate zwischen 50 und 2000 l Wasserstoff/l Charge liegt.

**6.** Verfahren gemäß einem der Ansprüche 4 bis 5, bei dem die zu behandelnde Verbindung aus der Gruppe gewählt ist, die gebildet wird durch die n-Alkane, die n-Alkylcycloalkane und die Verbindungen mit wenigstens einer aromatischen Gruppe.

**7.** Verfahren gemäß einer der Ansprüche 4 bis 6, bei dem die zu behandelnde Verbindung in einer Charge mit einem Start-Siedepunkt von mehr als 175°C präsent ist.

**8.** Verfahren gemäß einem der Ansprüche 4 bis 6, bei dem die zu behandelnde Verbindung in einer Charge mit einem Start-Siedepunkt von wenigstens 380°C präsent ist.

**9.** Verfahren gemäß einem der Ansprüche 4 bis 8, bei dem die zu behandelnde Verbindung in einer Kohlenwasserstoffcharge präsent ist, die aus der Gruppe gewählt ist, die gebildet wird durch die Mitteldestillate, die Vakuumrückstände, die Hydrocrack-Rückstände, die aus dem Fischer-Tropsch-Verfahren hervorgehenden Paraffine, die Syntheseöle, die Gasölschnitte, die Mitteldestillate aus FCC, den Grundstoffen für Öle, die Polyalphaolefine.

**Claims**

**1.** A catalyst comprising a matrix, at least 5% by weight of at least one hydro-dehydrogenating element selected from the group formed by non noble GVIII metals, GVIB metals, and niobium, and at least one molecular sieve with a one- or two-dimensional pore network, the accessible pore openings of which being delimited by 10 oxygen atoms, and a distance termed the bridging distance between the pores of less than 0.70 nm, and said sieve being selected from the group formed by NU-10, NU-23, NU-87, Theta-1 and EU-13, and said sieve containing atoms of boron, gallium and/or zinc in the zeolitic framework, and the zeolite is such that the catalyst constituted by the zeolite and 0.5% by weight of platinum, when subjected to the standard n-heptadecane isomerisation test has a selectivity for isomerised products of at least 70%, for a conversion of 95%.

2. A catalyst according to claim 1, also comprising phosphorous.

3. A catalyst according to claim 1 or claim 2, also containing sulphur.

4. A process for selective hydroisomerisation of compounds containing at least one n-alkane chain containing more than 10 carbon atoms in which said compound is brought into contact with a catalyst according to any one of claims 1 to 3.

5. A process according to claim 4 in which the pressure is in the range 1 to 250 bars, the temperature is in the range 170°C to 500°C, the hourly space velocity is in the range 0.05 to 100 h$^{-1}$, and the hydrogen flow rate is in the range 50 to 2000 l of hydrogen /l of feed.

6. A process according to claim 4 or claim 5, in which the compound to be treated is selected from the group formed by n-alkanes, n-alkylcycloalkanes and compounds containing at least one aromatic group.

7. A process according to any one of claims 4 to 6, in which the compound to be treated is present in a feed with an initial boiling point of more than 175°C.

8. A process according to any one of claims 4 to 6, in which the compound to be treated is present in a feed with an initial boiling point of at least 380°C.

9. A process according to any one of claims 4 to 8, in which the compound to be treated is present in a hydrocarbon feed selected from the group formed by middle distillates, vacuum residues, hydrocracking residues, paraffins from the Fischer-Tropsch process, synthesis oils, gas oil cuts, FCC middle distillates, lubricant stock and polyal-phaolefins.